# EUROPEAN PATENT APPLICATION

(11) **EP 4 512 467 A1**
(43) Date of publication of application: **26.02.2025**
(21) Application number: 23201196.5
(22) Date of filing: 02.10.2023
(51) Int. Cl.: A61N 5/10

(54) **DETERMINING A GATING WINDOW FOR DELIVERING A BEAM OF THERAPEUTIC RADIATION**

(30) Priority: 22.08.2023 US 202363533969 P
(71) Applicant: Koninklijke Philips N.V., 5656 AG Eindhoven (NL)
(72) Inventor: KABUS, Sven, Eindhoven (NL); COVITCH, Adam, Eindhoven (NL); KRUIS, Matthijs Ferdinand, 5656AG Eindhoven (NL); PERKINS, Amy, Eindhoven (NL); BAL, Matthieu Frédéric, Eindhoven (NL)
(74) Representative: Philips Intellectual Property & Standards

(57) **Abstract**

A system (100) for determining a gating window (110) for delivering a beam of therapeutic radiation to an anatomical region (130) undergoing cyclical motion, is provided. The system includes one or more processors (140) configured to determine, for each of one or more sets of at least one constraint (Dₘₐₓ) imposed on the anatomical region, a maximum gating window (110) throughout which a position of the anatomical region (130) in a temporal sequence of images (160_{1..m}) satisfies the set of at least one constraint, and a corresponding start (Ampₛₜₐᵣₜ, Pₛₜₐᵣₜ) and end (Amp_{end}, P_{end}) of the gating window in the cycle.

## Description

### TECHNICAL FIELD

The present disclosure relates to determining a gating window for delivering a beam of therapeutic radiation to an anatomical region undergoing cyclical motion. A system, a computer-implemented method, and a computer program product, are disclosed.

### BACKGROUND

External beam radiation therapy is a form of treatment in which an external source of radiation is delivered to anatomical regions in order to damage cancerous cells, and thereby stop them from growing or spreading to other parts of the anatomy. Various types of radiation may be delivered in this manner, including X-ray radiation, proton radiation, and electron beam radiation. The radiation is delivered in the form of one or more beams in order to provide a target dose to a target anatomical region whilst minimizing the dose that is delivered to healthy anatomical regions, some of which are referred-to as "organs at risk". The target anatomical region is often treated by aiming beams of radiation at the anatomical region from different directions so that their combined dose distribution conforms to the shape of the target anatomical region.

Prior to the delivery of the radiation therapy, a treatment planning stage is performed during which medical images of the anatomy are analyzed in order to determine how best to deliver the beam(s) of radiation to the target anatomical region. Treatment planning typically starts with an initial step of segmenting the medical images in order to identify the target anatomical regions and the organs at risk. Treatment goals are then specified. Treatment goals include objectives, which may specify requirements for the radiation dose delivered to parts of the anatomical region, which should be fulfilled, and/or constraints for the radiation dose delivered to parts of the anatomical region, which must be fulfilled. The constraints and/or objectives are then placed on the dose that should be delivered to each type of region. An optimization procedure is then performed wherein the multi-leaf collimator "MLC" leaf positions that control the spatial distribution of the dose that is delivered by each beam, are simulated in order to determine optimal "MLC" leaf positions that meet the constraints and best meets the objectives. The direction and/or angle of each beam may also be optimized during the optimization procedure.

In order to ensure that the constraints and/or objectives are met in spite of any motion of the anatomical regions during the subsequent delivery of the radiation therapy, margins are typically placed around the target anatomical region. For instance, a so-called "planning volume" that encompasses the expected positions of a tumor in all respiratory states may be defined. During treatment planning, the optimization procedure takes account of such margins. For instance, a constraint may ensure that the target dose is delivered to the planning volume that encompasses a tumor, rather than to the delineated tumor alone. A drawback of simply applying such margins to target anatomical regions in this manner is that it increases the dose that is delivered to tissue outside the target anatomical region.

In order to address this issue, various gating strategies have been developed. Gating is a technique wherein the beam(s) of radiation are only switched on during a period known as a "gating window" and in which the target anatomical region is expected to be in a known position. For instance, respiratory gating is a technique in which motion of the surface of the chest is tracked, e.g. via a video camera that tracks a position of an external marker on the chest, or via a strain sensor that is incorporated into a chest belt, in order to derive a respiratory motion signal. The respiratory motion signal is then used to select a period in which the motion of the anatomical region is expected to be minimal, for example around the end of the exhale state. As compared to using a planning volume that encompasses the expected positions of a tumor in all respiratory states and delivering a beam without gating, the use of the gating window facilitates the use of a relatively smaller planning volume during treatment planning. This reduces the dose to the tissue outside of the target anatomical region at the expense of increased treatment time.

However, such gating strategies rely upon the accuracy of the motion of the surface of the chest as a surrogate for the motion of the underlying anatomical region to which the beam(s) of radiation are delivered. In practice, there may only be a weak correlation between the motion of the surface of the chest, and the motion of the underlying anatomical region. For instance, the phase of the motion of the underlying anatomical region may lag behind the phase of a surrogate motion signal that is generated by tracking the motion of the surface of the chest using an external marker and a camera. The underlying anatomical region may also deform during the motion. As a result, the treatment planning for some patients is performed with a conservative planning volume and a relatively short gating window, resulting in an unnecessarily long treatment duration. The treatment planning for other patients is performed by simply enlarging the planning volume such that it encompasses all respiratory states, and the beam is delivered without gating. This results in an unnecessarily high dose to healthy tissue. In other words, current gating strategies can result in the generation of sub-optimal treatment plans.

Consequently, there is a need to support physicians in assessing the clinical benefits of different gating strategies when planning the delivery of radiation therapy.

A document WO 00/24467 A1 discloses a method and system for physiological gating for radiation therapy. An optical or video image system is used to measure regular physiological movement of a patient's body. The image data can be used to quantify voluntary or involuntary motion of the patient that may affect the delivery of radiation to a target valve. A gating signal can be generated to suspend delivery of radiation upon certain threshold event detected in the motion cycle.

### SUMMARY

According to a first aspect of the present disclosure, a system for determining a gating window for delivering a beam of therapeutic radiation to an anatomical region undergoing cyclical motion, is provided. The system includes one or more processors configured to:
receive medical image data, the medical image data comprising a temporal sequence of images representing the cyclical motion of the anatomical region;
determine, for each of one or more sets of at least one constraint imposed on the anatomical region, a maximum gating window throughout which a position of the anatomical region in the images in the temporal sequence satisfies the set of at least one constraint, and a corresponding start and end of the gating window in the cycle; and
output an indication of the one or more maximum gating windows, and for each maximum gating window, the corresponding start and end of the gating window in the cycle.

In this aspect, the system determines the maximum gating window throughout which a position of the anatomical region in the images in the temporal sequence satisfies the set of at least one constraint, it is ensured that the gating window has the maximum duration for meeting the set of at least one constraint. This helps to minimize the duration of the treatment time. Moreover, since the maximum gating window is determined using the temporal sequence of images, it is determined based on the actual motion of the anatomical region. It is therefore not reliant on an assumed correlation between the motion of the surface of the chest, and the motion of the underlying anatomical region to which the beam of radiation is delivered. The system therefore provides a more reliable calculation of the maximum gating window. This in turn enables a physician to perform a more accurate assessment of the clinical benefits of using the gating window.

According to a second aspect of the present disclosure, a system for quantifying a displacement of an anatomical region undergoing cyclical motion, is provided. The system comprising one or more processors configured to:
receive medical image data, the medical image data comprising a temporal sequence of images representing the displacement of the anatomical region during the cyclical motion;
determine, for each of one or more gating windows during a cycle of the cyclical motion, a corresponding start and end of the gating window defining a portion of the cycle throughout which a size of an envelope swept by the anatomical region in the images in the temporal sequence is smallest for the duration of the gating window; and
output an indication of the one or more gating windows, and for each gating window, an indication of the corresponding start and end of the gating window in the cycle and an indication of the size of the envelope swept by the anatomical region.

In this aspect, the system quantifies the displacement of the anatomical region by outputting an indication of the size of the envelope swept by the anatomical region for each gating window. Since each gating window defines a portion of the cycle throughout which a size of an envelope swept by the anatomical region in the images in the temporal sequence is smallest for the duration of the gating window, the system determines the minimal-motion portion of the cycle for the duration of the gating window. Since the maximum gating window is determined using the temporal sequence of images, it is determined based on the actual motion of the anatomical region. It is therefore not reliant on an assumed correlation between the motion of the surface of the chest, and the motion of the underlying anatomical region to which the beam of radiation is delivered. The system therefore provides a more reliable calculation of the gating window. This in turn enables a physician to perform a more accurate assessment of the clinical benefits of using the gating window.

According to a third aspect of the present disclosure, a system for generating a treatment plan for delivering a plurality of beams of therapeutic radiation to an anatomical region undergoing cyclical motion, is provided. The system includes one or more processors configured to:
receive medical image data, the medical image data comprising a temporal sequence of images representing the cyclical motion of the anatomical region;
receive constraint and/or objective data defining a set of at least one constraint and/or objective for the anatomical region; and
generate a first treatment plan for the anatomical region based on the medical image data and the constraint data and/or objective data; and
wherein the generating a first treatment plan comprises, for each of the beams of therapeutic radiation, simultaneously optimizing a gating window and a corresponding set of multi-leaf collimator leaf positions for delivering the beam of therapeutic radiation to the anatomical region in the temporal sequence of images so as to meet the set of at least one constraint and/or objective and to maximize a duration of the gating window.

In this aspect, the system simultaneously optimizes the gating window and the corresponding set of multi-leaf collimator leaf positions for delivering the beam of therapeutic radiation to the anatomical region in the temporal sequence of images so as to meet the set of at least one constraint and/or objective and to maximize a duration of the gating window. Simultaneously optimizing these parameters provides a more optimal combination of the gating window, and the corresponding set of multi-leaf collimator leaf positions, over e.g. sequentially optimizing these parameters.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for determining a gating window for delivering a beam 120 of therapeutic radiation to an anatomical region 130 undergoing cyclical motion, in accordance with some aspects of the present disclosure.
Fig. 2 is a flowchart illustrating an example of a computer-implemented method of determining a gating window for delivering a beam of therapeutic radiation to an anatomical region undergoing cyclical motion, in accordance with some aspects of the present disclosure.

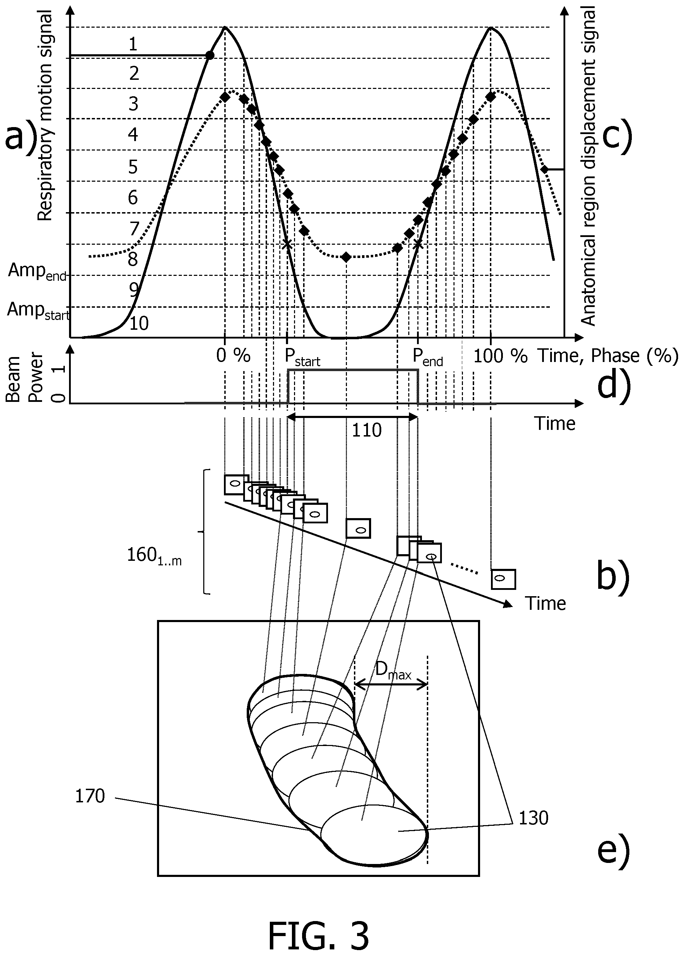

Fig. 3 illustrates: a) an example of a respiratory motion signal representing the cyclical motion of an extra-corporeal fiducial marker, b) an example of a temporal sequence of amplitude-gated images 160_{1..m} representing the cyclical motion of an anatomical region 130, c) an example of an anatomical region displacement signal derived from the temporal sequence of amplitude-gated images 160_{1..m}, d) an example of a gating window 110, and e) an example of an envelope 170 swept by the anatomical region 130 in the temporal sequence of amplitude-gated images 160_{1..m} during the gating window 110, in accordance with some aspects of the present disclosure.
Fig. 4 illustrates: a) an example of a respiratory motion signal representing the cyclical motion of an extra-corporeal fiducial marker, b) an example of an anatomical region displacement signal derived from a temporal sequence of phase-gated images, and c) an example of a gating window 110, in accordance with some aspects of the present disclosure.
Fig. 5 is a flowchart illustrating an example of a computer-implemented method of quantifying a displacement of an anatomical region undergoing cyclical motion, in accordance with some aspects of the present disclosure.
Fig. 6 illustrates: a) an example of a respiratory motion signal representing the cyclical motion of an extra-corporeal fiducial marker, b) an example of an anatomical region displacement signal derived from a temporal sequence of phase-gated images, and c) examples of gating windows 110_{1..6}, in accordance with some aspects of the present disclosure.
Fig. 7 is a flowchart illustrating an example of a computer-implemented method of generating a treatment plan for delivering a plurality of beams of therapeutic radiation to an anatomical region undergoing cyclical motion, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to examples of a system for determining a gating window for delivering a beam of therapeutic radiation to an anatomical region undergoing cyclical motion. Reference is also made to examples of a system for quantifying a displacement of an anatomical region undergoing cyclical motion. Reference is also made to examples of a system for generating a treatment plan for delivering a plurality of beams of therapeutic radiation to an anatomical region undergoing cyclical motion. In these example systems, reference is made to examples in which the cyclical motion arises from respiratory motion. It is however, to be appreciated that the cyclical motion may alternatively arise from other types of motion, including cardiac motion, for example. In these example systems, reference is also made to examples in which the anatomical region undergoing the cyclical motion is a tumor in a lung. However, it is also to be appreciated that the anatomical region may in general be any anatomical region. It is also to be appreciated that the beams of therapeutic radiation referred-to in these example systems may in general be any type of therapeutic radiation, such as X-ray radiation, proton radiation, and electron beam radiation, for example.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some of the operations that are performed by the one or more processors of the systems disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there is a need to support physicians in assessing the clinical benefits of different gating strategies when planning the delivery of radiation therapy.

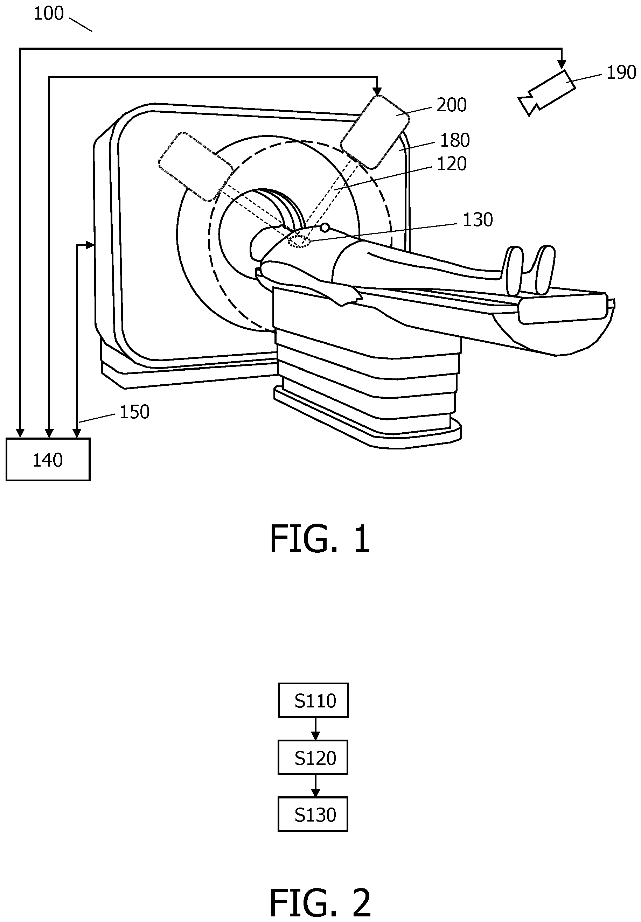

Fig. 1 is a schematic diagram illustrating an example of a system 100 for determining a gating window for delivering a beam 120 of therapeutic radiation to an anatomical region 130 undergoing cyclical motion, in accordance with some aspects of the present disclosure. Fig. 2 is a flowchart illustrating an example of a computer-implemented method of determining a gating window for delivering a beam of therapeutic radiation to an anatomical region undergoing cyclical motion, in accordance with some aspects of the present disclosure. It is noted that operations described as being performed by the one or more processors 140 of the system 100 illustrated in Fig. 1, may also be performed in the method illustrated in Fig. 2. Likewise, operations described in relation to the method illustrated in Fig. 2, may also be performed by the one or more processors 140 of the system 100 illustrated in Fig. 1.

With reference to Fig. 1, the system 100 for determining a gating window 110 for delivering a beam 120 of therapeutic radiation to an anatomical region 130 undergoing cyclical motion, include one or more processors 140 configured to:
receive S110 medical image data 150, the medical image data 150 comprising a temporal sequence of images 160_{1..m} representing the cyclical motion of the anatomical region 130;
determine S120, for each of one or more sets of at least one constraint imposed on the anatomical region, a maximum gating window 110 throughout which a position of the anatomical region 130 in the images 160_{1..m} in the temporal sequence satisfies the set of at least one constraint, and a corresponding start Ampₛₜₐᵣₜ, Pₛₜₐᵣₜ and end Amp_{end}, P_{end} of the gating window in the cycle; and
output S130 an indication of the one or more maximum gating windows 110, and for each maximum gating window, the corresponding start Ampₛₜₐᵣₜ, Pₛₜₐᵣₜ and end Amp_{end}, P_{end} of the gating window in the cycle.

In this aspect, the system 100 determines the maximum gating window throughout which a position of the anatomical region in the images in the temporal sequence satisfies the set of at least one constraint, it is ensured that the gating window has the maximum duration for meeting the set of at least one constraint. This helps to minimize the duration of the treatment time. Moreover, since the maximum gating window is determined using the temporal sequence of images, it is determined based on the actual motion of the anatomical region. It is therefore not reliant on an assumed correlation between the motion of the surface of the chest, and the motion of the underlying anatomical region to which the beam of radiation is delivered. The system therefore provides a more reliable calculation of the maximum gating window. This in turn enables a physician to perform a more accurate assessment of the clinical benefits of using the gating window.

With reference to the system 100, illustrated in Fig. 1, in the operation S110, the one or more processors 140 receive medical image data 150. The medical image data 150 comprises a temporal sequence of images 160_{1..m} representing the cyclical motion of the anatomical region 130.

The medical image data 150 that is received in the operation S 110 may be generated by various types of imaging systems. These include imaging systems that generate medical image data that can be reconstructed into a volumetric image, i.e. volumetric medical image data, and also imaging systems that generate medical image data that can be reconstructed into a two-dimensional image, i.e. projection medical image data. For instance, the medical image data 150 may be generated by a magnetic resonance imaging "MRI" system, or by a computed tomography "CT" imaging system, or by an ultrasound imaging system, or by a projection X-ray imaging system.

In the example illustrated in Fig. 1, the medical image data 150 is generated by a CT imaging system. In examples in which the medical image data is generated by a CT, or a projection X-ray, imaging system, the imaging system may be spectral X-ray imaging system. In other words, the medical image data 150 may be generated by a spectral CT imaging system, or a spectral X-ray projection imaging system. Spectral X-ray imaging systems generate X-ray attenuation data representing X-ray attenuation within multiple different energy intervals. The X-ray attenuation data from spectral X-ray imaging systems may be processed in order to distinguish between media that have similar X-ray attenuation values when measured within a single energy interval, and which would be indistinguishable in conventional X-ray attenuation data. Thus, the X-ray attenuation data from spectral X-ray imaging systems may be used to provide images with improved specificity to materials such as contrast agent, tissue, bone, and so forth.

The medical image data 150 that is received in the operation S 110 comprises a temporal sequence of images 160_{1..m}. In some examples described below, the images in the temporal sequence are generated using an amplitude-gating technique wherein each image in the temporal sequence is acquired in response to the value of a motion signal traversing a threshold value. In other examples described below, the images in the temporal sequence are generated using a phase-gating technique wherein each image in the temporal sequence is acquired in response to the phase of a motion signal traversing a threshold phase value. The threshold phase value may for instance be a threshold respiratory phase value, such as a proportion of the respiratory phase. The temporal sequence of images may alternatively be generated in the absence of such gating techniques, for example by generating the images at regular time intervals.

In general, the medical image data 150 that is received in the operation S110 may be raw data, i.e. data that has not yet been reconstructed into an image, or it may be reconstructed image data, i.e. data that has already been reconstructed into an image.

The medical image data 150 that is received in the operation S 110 may be received from various sources, including from a medical imaging system such as the imaging systems described above. In the example illustrated in Fig. 1, the medical image data 150 is received from a CT imaging system 180. Alternatively, the medical image data 150 may be received from another source, such as a database, a computer readable storage medium, the internet, the cloud, and so forth. The medical image data 150 may be received via any form of data communication, including wired and wireless communication. By way of some examples, when wired communication is used, the communication may take place via an electrical or optical cable, and when wireless communication is used, the communication may for example be via RF or infrared signals.

The medical image data 150 that is received in the operation S110 represents the cyclical motion of the anatomical region 130. In general, the anatomical region may be any anatomical region. The anatomical region may be a tumor in a lung, for example. In general, the cyclical motion may arise from any type of motion that is cyclical in nature. The cyclical motion may arise from respiratory motion, or cardiac motion, for example.

Returning to the system illustrated in Fig. 1, in the operation S120, the one or more processors 140 determine, for each of one or more sets of at least one constraint imposed on the anatomical region, a maximum gating window 110 throughout which a position of the anatomical region 130 in the images 160_{1..m} in the temporal sequence satisfies the set of at least one constraint, and a corresponding start Ampₛₜₐᵣₜ, Pₛₜₐᵣₜ and end Amp_{end}, P_{end} of the gating window in the cycle.

In general, a gating window 110 defines a portion of the cycle of the motion. The gating window may for instance include a range of phase values in the cycle, or a range of amplitude values in the cycle. In general, each maximum gating window is determined by using image processing techniques to track the position of the anatomical region 130 in the images 160_{1..m} in the temporal sequence whilst applying the corresponding set of constraint(s). In some examples, the operation S120 may include segmenting the images 160_{1..m} in the temporal sequence in order to identify the anatomical region 130. Various segmentation techniques may be used for this purpose, including region growing, thresholding, feature detection, model-based segmentation. A neural network such as a U-Net may also be trained to segment the anatomical region.

In general, the constraint(s) that are imposed on the anatomical region 130 in the operation S120 may include dose and/or displacement constraints. Examples of the constraint(s) that are imposed on the anatomical region 130 in the operation S120 include: a constraint requiring that a displacement of the anatomical region 130 remains less than a specified value; a constraint requiring that the anatomical region 130 in the images in the temporal sequence remains within a specified boundary; a constraint requiring that a specified portion of the anatomical region 130 remains within a specified boundary for a specified portion of the cycle; and a constraint requiring that the anatomical region 130 receives a specified dose from a specified dose distribution of the beam 120 of therapeutic radiation.

The constraint(s) that are imposed on the anatomical region 130 in the operation S 120 may be received by the one or more processors 140 in the form of constraint data. The constraint data may be generated automatically, or it may be generated in response to user input. In the former case, the constraint data may be provided automatically by the one or more processors in the form of recommended values. In the latter case, a user may select the constraint(s) from a list of items displayed in a menu, or the user may define the constraint(s) in an image representing the anatomical region. For instance, a user may define a constraint requiring that the displacement of the anatomical region 130 remains less than a specified value by selecting the specified value from a menu, or by delineating the maximum permissible extent of the displacement in an image representing the anatomical region.

In addition to determining a maximum gating window 110 for each set of constraint(s), the operation S 120 also includes determining a start and an end of each maximum gating window in the cycle, i.e. Ampₛₜₐᵣₜ and Amp_{end}, or alternatively Pₛₜₐᵣₜ and P_{end}.

In general, the start and the end of each maximum gating window is determined in a motion signal representing the cyclical motion of the anatomical region 130. In one example, the motion signal may be received from a motion sensor configured to monitor the cyclical motion of the anatomical region 130. In this case, the motion signal is generated contemporaneously with the medical image data 150. The motion sensor may for instance include a video camera. The video camera may track a position of a fiducial marker that is attached to an exterior of the anatomical region. Image processing techniques may be applied to image data generated by the video camera in order to extract the motion signal. In this case, the start Ampₛₜₐᵣₜ, or Pₛₜₐᵣₜ, and the end Amp_{end}, or P_{end}, of each maximum gating window may be defined in the motion signal that is extracted from the image data. Alternatively, other types of motion sensors such as respiration monitor belt, an accelerometer, and so forth, may provide the motion signal. In another example, the motion signal may be extracted from the received medical image data 150. In this example, image processing techniques may be applied to the temporal sequence of images in the medical image data 150 in order to determine the position of a centroid, or another portion of the anatomical region, over time. In this case, the start Ampₛₜₐᵣₜ or Pₛₜₐᵣₜ, and the end Amp_{end} or P_{end}, of each maximum gating window may be defined in the motion signal that is extracted from the medical image data 150.

Various techniques are contemplated for determining the maximum gating window(s) 110 in the operation S 120. In general, these include so-called brute-force techniques, and optimization techniques. In brute-force techniques, an optimal solution is determined by evaluating an objective function for all possible input parameters and selecting the input parameters that provide the optimal value of the objective function. In optimization techniques, rather than evaluating the objective function for all possible input parameters, the input parameters are adapted iteratively based on the value of the objective function using techniques such as Gradient Descent.

In one example, the one or more processors 140 are configured to determine the one or more maximum gating windows 110 by:
determining a shape of an envelope 170 swept by the anatomical region 130 in the images 160_{1..m} in the temporal sequence corresponding to the gating window 110; and
assessing the shape of the envelope with respect to the set of at least one constraint.

In this example, the shape of the envelope encompasses the positions that have been occupied by the anatomical region throughout the duration of the gating window. The shape of the envelope also encompasses deformations of the anatomical region throughout the duration of the gating window. Consequently, the assessment of the shape of the envelope with respect to the set of at least one constraint facilitates an accurate evaluation of whether or not the constraint(s) are met.

This example is now described with reference to Fig. 3, which illustrates: a) an example of a respiratory motion signal representing the cyclical motion of an extra-corporeal fiducial marker, b) an example of a temporal sequence of amplitude-gated images 160_{1..m} representing the cyclical motion of an anatomical region 130, c) an example of an anatomical region displacement signal derived from the temporal sequence of amplitude-gated images 160_{1..m}, d) an example of a gating window 110, and e) an example of an envelope 170 swept by the anatomical region 130 in the temporal sequence of amplitude-gated images 160_{1..m} during the gating window 110, in accordance with some aspects of the present disclosure.

The respiratory motion signal illustrated in Fig. 3a) may be obtained by tracking the position of an extra-corporeal fiducial marker that is attached to an exterior of the anatomical region using a video camera 190. As illustrated in Fig. 1, the video camera 190 is configured to view the exterior of the anatomical region during the acquisition of the medical image data 150 by the CT imaging system 180. Image processing techniques may be applied to image data generated by the video camera in order to extract the respiratory motion signal illustrated in Fig. 3a). Alternative techniques for providing a motion signal are described above. The temporal sequence of amplitude-gated images 160_{1..m} illustrated in Fig. 3b) is generated contemporaneously with the medical image data 150. Each amplitude-gated image 160_{1..m} is acquired in response to the value of the respiratory motion signal illustrated in Fig. 3a) signal traversing a threshold value. In the example illustrated in Fig. 3a) there are ten equally-spaced amplitude threshold values labelled 1..10, and an image 160 is acquired by the CT imaging system 180 when the value of the respiratory motion signal traverses each threshold value. As illustrated in the temporal sequence of images 160_{1..m} in Fig. 3b), this typically results in the acquisition of the images 160_{1..m} at irregular time intervals.

The anatomical region displacement signal illustrated in Fig. 3c) is derived from the temporal sequence of amplitude-gated images 160_{1..m} by tracking the position of the centroid of the anatomical region in the images 160_{1..m} illustrated in Fig. 3b). In the example illustrated in Fig. 3, the anatomical region displacement signal illustrated in Fig. 3c) is calculated as the Euclidian distance from a reference position. The anatomical region displacement signal illustrated in Fig. 3c) may alternatively be derived by tracking a position of another point, or a portion of the anatomical region, in the images 160_{1..m} illustrated in Fig. 3b). It is noted that the anatomical region displacement signal illustrated in Fig. 3c) represents the actual motion of the anatomical region, whereas the respiratory motion signal illustrated in Fig. 3a) serves as a surrogate for this motion. It is also noted that there can be discrepancies between these signals. For instance, as may be observed from the signals illustrated in Fig. 3a and Fig. 3c), there may be time lag between the respiratory motion signal illustrated in Fig. 3a), and the anatomical region displacement signal illustrated in Fig. 3c).

The example gating window 110 illustrated in Fig. 3d) defines a portion of the respiratory motion signal illustrated in Fig. 3a) during which the beam of radiation is switched on. The envelope 170 swept by the anatomical region 130 in the temporal sequence of amplitude-gated images 160_{1..m} during the gating window 110 illustrated in Fig. 3d), is illustrated in Fig 3e). The gating window 110 in this example is the maximum gating window during which the position of the anatomical region 130) in the images 160_{1..m}) in the temporal sequence, as determined from the shape of the envelope 170, satisfies the example constraint that the displacement of the right-hand edge of the anatomical region 130 is less than the predetermined value Dₘₐₓ. In the example gating window 110 illustrated in Fig. 3d), the constraint is met, and also the gating window has a maximum duration, for the images 160_{8..14} in the temporal sequence. In the example illustrated in Fig. 3, the start Ampₛₜₐᵣₜ, or Pₛₜₐᵣₜ, and the end Amp_{end}, or P_{end}, of the gating window in the cycle, are determined in the respiratory motion signal. This enables the amplitude intervals of the respiratory motion signal to be used to trigger the gating of the beam during the subsequent delivery of the therapy. Thus, in the example illustrated in Fig. 3, the beam would be switched on when the respiratory motion signal falls below the threshold value Ampₛₜₐᵣₜ, i.e. between the 7^{th} and 8^{th} amplitude intervals, or at a point in time, Pₛₜₐᵣₜ, and the beam would be switched off when the respiratory motion signal subsequently exceeds the threshold value Amp_{end}, i.e. between the 7^{th} and 8^{th} amplitude intervals, or at a point in time, P_{end}. In other words, the beam is on whilst the value of the respiratory motion signal is less than 30% of its maximum amplitude, i.e. during the period between the cross symbols marked on the respiratory motion signal.

The shape of the envelope in this example may be calculated in various ways. In one example, the shape of the envelope is determined by segmenting the images in the temporal sequence in order to determine the boundary of the anatomical region, and calculating displacement vector fields that represent the displacement of the boundary of the anatomical region between pairs of images in the temporal sequence. The shape of the envelope may represent the area, or the volume, swept by the boundary of the anatomical region over the duration of the gating window, as illustrated in Fig. 3e). The shape of the envelope in this example may be calculated over a period corresponding to the exact time interval between pairs of images, as illustrated in Fig. 3e), or, alternatively the shape of the envelope may be calculated over a different time interval by interpolating the position of the anatomical region to provide its position at a point in time between the times of the pair of images. This may be useful in improving the temporal resolution at which the shape of the envelope is calculated, and consequently in providing a gating window having a duration that does not exactly correspond to the duration of a series of consecutive image in the temporal sequence.

An example of an alternative technique for evaluating the envelope is to segment the images as described above, and to perform a maximum intensity projection on the anatomical region over the duration of the gating window.

Using the images 160_{1..m} in the temporal sequence to determine the gating window 110 in the above-described manner is reliable because the images 160_{1..m} reflect the actual motion of the anatomical region 170. If instead, the respiratory motion signal illustrated in Fig. 3a) were used to determine the gating window 110, the phase lag between the actual motion of the anatomical region, and the surrogate motion provided by the respiratory motion signal, might have resulted in a gating window with a sub-optimal duration, or a sub-optimal start and/or end. Moreover, the respiratory motion signal illustrated in Fig. 3a) contains no information about the deformation of the anatomical region over the course of the gating window 110. Thus, if the respiratory motion signal illustrated in Fig. 3a) were used to determine the gating window 110, this might also have resulted in a gating window with a sub-optimal duration, or a sub-optimal start and/or end.

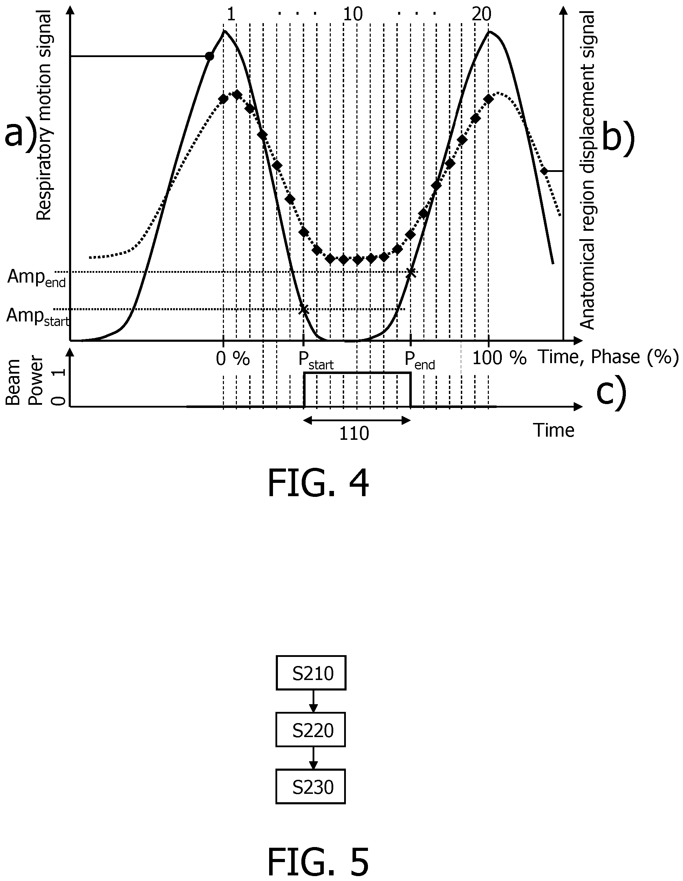

In the example described above with reference to Fig. 3, the temporal sequence of images 160_{1..m} illustrated in Fig. 3b) was generated using amplitude-gating. It is noted that the temporal sequence of images 160_{1..m} may alternatively be generated using phase-gating. An example in which the temporal sequence of images 160_{1..m} is generated using phase-gating is now described with reference to Fig. 4, which illustrates: a) an example of a respiratory motion signal representing the cyclical motion of an extra-corporeal fiducial marker, b) an example of an anatomical region displacement signal derived from a temporal sequence of phase-gated images, and c) an example of a gating window 110, in accordance with some aspects of the present disclosure. The respiratory motion signal illustrated in Fig. 4a corresponds to the respiratory motion signal illustrated in Fig. 3a, and is generated in the same manner. Referring to the respiratory motion signal illustrated in Fig. 4a, phase intervals 1..20 are defined in this signal at regular time intervals. The phase axis in Fig. 4a therefore also corresponds to time. The phase intervals 1..20 represent proportions of the respiratory cycle. In the phase-gating approach illustrated in Fig. 4, an image in the temporal sequence is acquired in response to the phase of the respiratory motion signal illustrated in Fig. 4a traversing each threshold phase value. This results in the acquisition of the images 160_{1..m} at regular phase intervals, each being 5% of the complete cycle, i.e. at regular time intervals.

The anatomical region displacement signal illustrated in Fig. 4b) is derived from the temporal sequence of phase-gated images 160_{1..m} in the same manner as that described above, i.e. by tracking the position of the centroid of the anatomical region in the phase-gated images. The example gating window 110 illustrated in Fig. 4c) defines a portion of the respiratory motion signal illustrated in Fig. 4a) during which the beam of radiation is switched on. The shape of the envelope swept by the anatomical region in the temporal sequence of phase-gated images 160_{1..m} may be determined in the same manner as described above and assessed with respect to the set of at least one constraint in order to determine the a maximum gating window 110 throughout which the position of the anatomical region 130 in the images 160_{1..m} in the temporal sequence satisfies the set of at least one constraint. In the example illustrated in Fig. 4, the start Ampₛₜₐᵣₜ, or Pₛₜₐᵣₜ, and end Amp_{end}, or P_{end}, of the gating window in the cycle, are determined in the respiratory motion signal. This enables the phase intervals of the respiratory motion signal to be used to trigger the gating of the beam during the subsequent delivery of the therapy. In the example illustrated in Fig. 4, the beam would be switched on when the phase of the respiratory motion signal is between the 30% and 70% of the complete cycle of the cyclical motion, i.e. during the period between the cross symbols marked on the respiratory motion signal.

An example of a brute-force implementation of this example is now described in which the size of an envelope 170 swept by the anatomical region 130 in the images is used to generate a list of subsets of increasing numbers of images in which the size of an envelope 170 swept by the anatomical region 130 is smallest among all possible selections of the total number of consecutive images from the temporal sequence. This example may be used with a temporal sequence of images that is acquired using phase gating. In this example, the one or more processors 140 are configured to determine the one or more maximum gating windows 110, by:
generating, from the temporal sequence of images 160_{1..m}, a list of candidate subsets of consecutive images, wherein each candidate subset comprises a total number of images, wherein the total number of images in each candidate subset increases throughout the list, and wherein each candidate subset in the list is generated by:
selecting from the temporal sequence the corresponding total number of consecutive images throughout which a size of an envelope 170 swept by the anatomical region 130 is smallest among all possible selections of the total number of consecutive images from the temporal sequence; and
selecting from the list of candidate subsets, a candidate subset having a maximum total number of images throughout which the anatomical region 130 meets the constraint, the maximum gating window 110 being defined based on a difference between a time of a first image in the selected candidate subset and a time of a last image in the selected candidate subset, and the corresponding start Ampₛₜₐᵣₜ, or Pₛₜₐᵣₜ, and the end Amp_{end}, or P_{end}, of the gating window 110 in the cycle for the maximum gating window 110 being defined based on the time of the first image in the selected candidate subset and the time of the last image in the selected candidate subset.

In another example, the maximum gating window(s) are determined by calculating a displacement of the anatomical region between pairs of images in the temporal sequence. In this example, the one or more processors 140 are configured to:
calculate, for each of a plurality of pairs of images 160_{1..m} in the temporal sequence, a displacement of the anatomical region 130 between the pair of images; and
wherein the one or more processors 140 are configured to determine the one or more maximum gating windows 110, using the calculated displacements.

In this example, the displacement of the anatomical region is calculated over a period that corresponds to the time interval between each pair of images. The displacement of the anatomical region may be calculated over a different period by interpolating the position of the anatomical region to provide its position at a point in time between the times of the pair of images. As described above, this may be useful in improving the temporal resolution at which the displacements are calculated. The displacements may be calculated in various ways. In some examples, the displacements may be calculated in the form of displacement vector fields. The displacement vector fields represent the displacement of the anatomical region, or a part thereof, such as for example the displacement of a centroid of the anatomical region.

In another example, the one or more processors 140 are configured to determine the one or more maximum gating windows 110, by:
selecting from the temporal sequence of images 160_{1..m}, a series of images having a maximum duration and throughout which the anatomical region 130 meets the set of at least one constraint, the maximum gating window 110 being defined based on a difference between a time of a first image in the series and a time of a last image in the series, and the corresponding start Ampₛₜₐᵣₜ, Pₛₜₐᵣₜ and end Amp_{end}, P_{end} of the maximum gating window 110 in the cycle being defined based on the time of the first image in the series and the time of the last image in the series.

In this example it is determined whether the anatomical region meets each set of at least one constraint in different series of the images in the temporal sequence of images 160_{1..m}. For instance, it may be determined whether or not the constraint is met in a series of two, or three, or more, consecutive images. The series of images having a maximum duration and throughout which the anatomical region 130 meets the set of at least one constraint, is then selected. This series may be identified using a brute-force technique, or an optimization technique.

In some cases the maximum gating window 110 may then be defined using the exact difference between a time of a first image in the series and a time of a last image in the series. However, a gating window with a longer duration and throughout which the anatomical region 130 meets the set of at least one constraint, might be achieved by using interpolation to determine the position and/or shape of the anatomical region at a point in time prior to the first image in the selected series, or at a point in time after the last image in the selected series.

In other words, the start Ampₛₜₐᵣₜ, or Pₛₜₐᵣₜ, and/or the end Amp_{end}, or P_{end}, of the gating window 110 may coincide with the time of an image in the temporal sequence, or alternatively, the start Ampₛₜₐᵣₜ, or Pₛₜₐᵣₜ, and/or the end Amp_{end}, or P_{end}, of the gating window 110 may occur between a pair of consecutive images in the temporal sequence. In the latter case, interpolation may be used to determine the start Ampₛₜₐᵣₜ, or Pₛₜₐᵣₜ, and/or the end Amp_{end}, or P_{end}, of the gating window 110 based on the position and/or shape of the anatomical region in the images.

Returning to the system 100, illustrated in Fig. 1, in the operation S130, the one or more processors 140 output S130 an indication of the one or more maximum gating windows 110, and for each maximum gating window, the corresponding start Ampₛₜₐᵣₜ, or Pₛₜₐᵣₜ, and end Amp_{end}, or P_{end}, of the gating window in the cycle. These indications may be outputted in various ways, including via a display, such as a monitor. Alternatively, they may be outputted in a different manner, such as to a printer, or to a computer readable storage medium, for example. The indication of the one or more maximum gating windows 110 may be outputted numerically, or graphically, for example. The indication of the corresponding start Ampₛₜₐᵣₜ, or Pₛₜₐᵣₜ, and end Amp_{end}, or P_{end}, of the gating window in the cycle, may be outputted by outputting a motion signal, such as one of the motion signals described above, and providing markers in the motion signal that correspond to the start Ampₛₜₐᵣₜ, Pₛₜₐᵣₜ and end Amp_{end}, P_{end} of the gating window, as illustrated above in Fig. 3 and Fig. 4.

Having determined the maximum gating window(s) and the corresponding start Ampₛₜₐᵣₜ, Pₛₜₐᵣₜ and end Amp_{end}, P_{end} of the gating window in the cycle as described above, the system may also provide one or more recommendations. In one example, the one or more processors 140 are further configured to:
output i) a recommendation to plan a gated delivery of a beam 120 of therapeutic radiation to the anatomical region 130, or ii) a recommendation to plan a non-gated delivery of a beam 120 of therapeutic radiation to the anatomical region 130, or iii) a recommended fractionation scheme for delivering of a beam of therapeutic radiation to the anatomical region 130, for each set of at least one constraint, based on the at least one constraint and the maximum gating window 110.

These recommendations may be generated using various techniques, including for instance using decision tree, or a look-up table, or a trained neural network, that respectively relates the recommendation to the at least one constraint and the maximum gating window 110. These recommendations may be determined based on expert knowledge that is recorded in the decision tree, and so forth. By way of an example, it may be recommended to plan a gated delivery of a beam 120 of therapeutic radiation to the anatomical region 130 if e.g. 98% of the tumor volume receives a dose of e.g. 60 Gy during 100% of beam-on time.

In a second aspect of the present disclosure, the system 100 illustrated in Fig. 1 is used to quantify a displacement of the anatomical region 130. In this second aspect, a system 100 for quantifying a displacement of an anatomical region 130 undergoing cyclical motion, comprises one or more processors 140 configured to:
receive S210 medical image data 150, the medical image data 150 comprising a temporal sequence of images 160_{1..m} representing the displacement of the anatomical region 130 during the cyclical motion;
determine S220, for each of one or more gating windows 110_{1..i} during a cycle of the cyclical motion, a corresponding start Ampₛₜₐᵣₜ₁, Pₛₜₐᵣₜ, and end Amp_{end1}, P_{end1} of the gating window 110 defining a portion of the cycle throughout which a size of an envelope 170 swept by the anatomical region 130 in the images in the temporal sequence is smallest for the duration of the gating window 110; and
output S230 an indication of the one or more gating windows 110_{1..i}, and for each gating window 110₁, an indication of the corresponding start Ampₛₜₐᵣₜ₁, Pₛₜₐᵣₜ, and end Amp_{end1}, P_{end1} of the gating window in the cycle and an indication of the size of the envelope 170 swept by the anatomical region 130.

In this second aspect, the system 100 quantifies the displacement of the anatomical region by outputting an indication of the size of the envelope swept by the anatomical region for each gating window. Since each gating window defines a portion of the cycle throughout which a size of an envelope swept by the anatomical region in the images in the temporal sequence is smallest for the duration of the gating window, the system determines the minimal-motion portion of the cycle for the duration of the gating window. Since the maximum gating window is determined using the temporal sequence of images, it is determined based on the actual motion of the anatomical region. It is therefore not reliant on an assumed correlation between the motion of the surface of the chest, and the motion of the underlying anatomical region to which the beam of radiation is delivered. The system therefore provides a more reliable calculation of the gating window. This in turn enables a physician to perform a more accurate assessment of the clinical benefits of using the gating window.

The operations performed by the system 100 in this second aspect are illustrated in Fig. 5, which is a flowchart illustrating an example of a computer-implemented method of quantifying a displacement of an anatomical region undergoing cyclical motion, in accordance with some aspects of the present disclosure. It is noted that operations described as being performed by the one or more processors 140 of the system 100 illustrated in Fig. 1 in relation to this second aspect, may also be performed in the method illustrated in Fig. 5. Likewise, operations described in relation to the method illustrated in Fig. 5, may also be performed by the one or more processors 140 of the system 100 illustrated in Fig. 1 as elements of this second aspect.

In this second aspect, the operation S210 includes receiving medical image data 150. This operation corresponds to the operation S 110 described above in relation to the first aspect.

In the operation S220, a start Ampₛₜₐᵣₜ₁, Pₛₜₐᵣₜ₁ and end Amp_{end1}, P_{end1} of each of one or more gating windows is determined. Each gating window 110 defines a portion of the cycle throughout which a size of an envelope 170 swept by the anatomical region 130 in the images in the temporal sequence is smallest for the duration of the gating window 110.

The operation S220 may be performed using techniques that are similar to those described in relation to the operation S120 above. Thus, image processing techniques may be used to determine the envelope 170 swept by the anatomical region in the images in the temporal sequence. The size of the envelope, i.e. the area, in 2D images, or the volume, in 3D images, of the envelope is then calculated. Brute-force, or optimization, techniques are then used to identify a series of images throughout which the envelope size is smallest for the duration of the gating window. The start Ampₛₜₐᵣₜ₁, Pₛₜₐᵣₜ₁ and end Amp_{end1}, P_{end1} of each gating window 110 is then determined. As described in the operation S120 above, the start Ampₛₜₐᵣₜ₁, Pₛₜₐᵣₜ₁ and end Amp_{end1}, P_{end1} of each gating window 110 may be determined in a motion signal representing the cyclical motion of the anatomical region 130. The motion signal may be received from a motion sensor configured to monitor the cyclical motion of the anatomical region 130, or alternatively, the motion signal may be extracted from the received medical image data 150.

In one example, the gating window(s) in this second aspect are determined by calculating a displacement of the anatomical region 130 between pairs of consecutive images. In this example, the one or more processors 140 are configured to:
calculate, for each of a plurality of pairs of images 160_{1..m} in the temporal sequence, a displacement of the anatomical region 130 between the pair of consecutive images; and
wherein the one or more processors 140 are configured to determine the one or more gating windows 110_{1..i}, using the calculated displacements.

In another example, in this second aspect, the one or more processors 140 are configured to determine the start Ampₛₜₐᵣₜ, Pₛₜₐᵣₜ and end Amp_{end}, P_{end} of the gating window 110 in the cycle for each of the one or more gating windows 110_{1..i} by determining a shape of an envelope 170 swept by the anatomical region 130 throughout each of a plurality of selections of images in the temporal sequence corresponding to the gating window, and determining the start Ampₛₜₐᵣₜ, Pₛₜₐᵣₜ and end Amp_{end}, P_{end} of the gating window based on the selection of images for which a corresponding size of the envelope 170 is smallest.

In another example, in this second aspect, the one or more processors 140 are configured to determine the start Ampₛₜₐᵣₜ, Pₛₜₐᵣₜ and end Amp_{end}, P_{end} of the gating window 110 in the cycle for each of the one or more gating windows 110_{1..i}, by:
selecting from the temporal sequence of images 160_{1..m}, a series of images having a duration corresponding to the gating window 110 and throughout which a size of an envelope 170 swept by the anatomical region 130 is smallest for the duration of the gating window, the corresponding start Ampₛₜₐᵣₜ, Pₛₜₐᵣₜ and end Amp_{end}, P_{end} of the gating window in the cycle being defined based on a time of a first image in the series and a time of a last image in the series.

In another example, in this second aspect, the one or more processors 140 are configured to determine the start Ampₛₜₐᵣₜ, Pₛₜₐᵣₜ and end Amp_{end}, P_{end} of the gating window 110 in the cycle for each of the one or more gating windows 110_{1..i}, by:
generating, from the temporal sequence of images 160_{1..m}, a list of candidate subsets of consecutive images, wherein each candidate subset comprises a total number of images, wherein the total number of images in each candidate subset increases throughout the list, and wherein each candidate subset in the list is generated by:
selecting from the temporal sequence the corresponding total number of consecutive images throughout which a size of an envelope 170 swept by the anatomical region 130 is smallest among all possible selections of the total number of consecutive images from the temporal sequence; and
selecting from the list of candidate subsets, a candidate subset having a total number of images corresponding to the gating window 110, the start Ampₛₜₐᵣₜ, Pₛₜₐᵣₜ and end Amp_{end}, P_{end} of the gating window in the cycle being defined based on a time of a first image in the selected candidate subset and a time of a last image in the selected candidate subset.

It is noted that in these examples, the start Ampₛₜₐᵣₜ, Pₛₜₐᵣₜ and/or the end Amp_{end}, P_{end} of the gating window 110 may coincide with the time of an image in the temporal sequence, or alternatively, the start Ampₛₜₐᵣₜ, Pₛₜₐᵣₜ and/or the end Amp_{end}, P_{end} of the gating window 110 may occur between a pair of consecutive images in the temporal sequence. In the latter case, interpolation may be used to determine the start Ampₛₜₐᵣₜ, Pₛₜₐᵣₜ and/or the end Amp_{end}, P_{end} of the gating window 110 based on the position and/or shape of the anatomical region in the images.

In the operation S230, an indication of the gating window(s), and an indication of the corresponding start Ampₛₜₐᵣₜ₁, Pₛₜₐᵣₜ₁ and end Amp_{end1}, P_{end1} of the gating window(s) in the cycle, and an indication of the size of the envelope 170 swept by the anatomical region 130, are outputted. These indications may be outputted in a similar manner to that described above in the operation S130. In one example, the one or more processors 140 are configured to output the indication of the size of the envelope 170, by:
outputting a graphical representation of the medical image data 150 including the anatomical region 130; and
providing the indication of the size of the envelope 170 in the graphical representation. In this example, the indication of the size of the envelope 170 may be provided numerically by labelling the anatomical region with the size, i.e. the area, or the volume, of the envelope 170, for example.

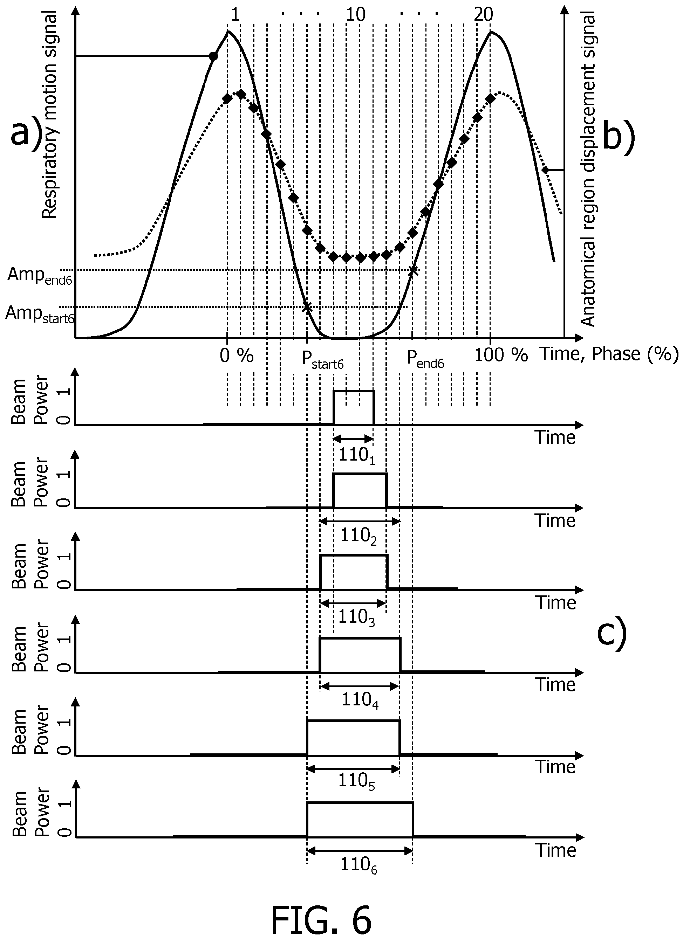

Examples of gating windows that are determined in accordance with this second aspect are now described with reference to Fig. 6, which illustrates: a) an example of a respiratory motion signal representing the cyclical motion of an extra-corporeal fiducial marker, b) an example of an anatomical region displacement signal derived from a temporal sequence of phase-gated images, and c) examples of gating windows 110_{1..6}, in accordance with some aspects of the present disclosure. The respiratory motion signal illustrated in Fig. 6a), and the anatomical region displacement signal illustrated in Fig. 6b), correspond to the same signals illustrated in Fig. 4, and a description of these signals will not be repeated in view of brevity. In brief, a temporal sequence of phase-gated images is acquired using the respiratory motion signal illustrated in Fig. 6a, i.e. an image in the temporal sequence is acquired in response to the phase of the respiratory motion signal illustrated in Fig. 6a traversing each of the threshold phase value labelled 1..10. These images are used to generate the anatomical region displacement signal illustrated in Fig. 6b), as described above with reference to Fig. 4. In Fig. 6c), six example gating windows 110_{1..6} are illustrated. In these examples the gating windows 110_{1..6} correspond to phase intervals having durations that are 15%, 20%, 25%, 30%, 35%, and 40% of a complete cycle of the cyclical motion, respectively. For each gating window, the system 100 determines a corresponding start and end of the gating window is determined in the respiratory motion signal. For example, the system 100 determines the start, Ampₛₜₐᵣₜ₆, Pₛₜₐᵣₜ₆, and the end Amp_{end6}, and P_{end6}, of the gating window 110₆ in the respiratory motion signal, as illustrated in Fig. 6a. The start, Ampₛₜₐᵣₜ₆, Pₛₜₐᵣₜ₆, and the end Amp_{end6}, and P_{end6}, of the gating window 110₆ correspond to the portion of the cycle throughout which a size of the envelope 170 swept by the anatomical region 130 in the images in the temporal sequence is smallest for a gating window 110₆ that represents 40% of a complete cycle of the cyclical motion. The gating window 110₆, may be used to gate the beam during the subsequent delivery of therapy. Thus, in the example illustrated in Fig. 6, the beam would be switched on for the gating window 110₆ when the phase of the respiratory motion signal reaches 30% of the complete cycle of the cyclical motion, and the beam would be switched off when the phase of the respiratory motion signal reaches 70% of the complete cycle of the cyclical motion, i.e. during the period between the cross symbols marked on the respiratory motion signal. Thus, in this second aspect, the system 100 provides the ability to determine optimal trigger points for different minimal-motion gating windows.

In this second aspect, the system 100 may also be used to investigate whether or not various constraints are met for each of the gating windows. Thus, in one example, the one or more processors 140 are configured to:
receive constraint data defining a set of at least one constraint for the anatomical region 130; and
for each of the one or more gating windows 110_{1..i}:
   evaluate a position of the anatomical region 130 with respect to the set of at least one constraint using the images in the temporal sequence corresponding to the gating window; and
   output an indication of whether or not the set of at least one constraint is met.

This enables a physician to assess the suitability of using a gating window for determining the plan. For instance, a physician may evaluate whether any of the constraints are too stringent. The physician may subsequently use this understanding to adjust a constraint that might otherwise inhibit the use of a gating window in generating a treatment plan.

In this second aspect, the system 100 may also provide one or more recommendations. In one example, the one or more processors 140 are configured to:
output i) a recommendation to plan a gated delivery of a beam 120 of therapeutic radiation to the anatomical region 130, or ii) a recommendation to plan a non-gated delivery of a beam 120 of therapeutic radiation to the anatomical region 130, or iii) a recommended fractionation scheme for delivering a beam 120 of therapeutic radiation to the anatomical region 130, for each of the one or more gating windows 110_{1..i}, based on the duration of the gating window and the size of the envelope 170 swept by the anatomical region 130.

These recommendations may be determined based on the duration of the gating window and the size of the envelope 170 swept by the anatomical region 130, using the techniques described above in relation to the second aspect.

In a third aspect of the present disclosure, the system 100 illustrated in Fig. 1 is used to generate a treatment plan for delivering a plurality of beams of therapeutic radiation to an anatomical region. In this third aspect, a system 100 for generating a treatment plan for delivering a plurality of beams 120 of therapeutic radiation to an anatomical region 130 undergoing cyclical motion, comprises one or more processors 140 configured to:
receive S310 medical image data 150, the medical image data 150 comprising a temporal sequence of images 160_{1..m} representing the cyclical motion of the anatomical region 130;
receive S320 constraint data and/or objective data defining a set of at least one constraint and/or objective for the anatomical region 130; and
generate S330 a first treatment plan for the anatomical region 130 based on the medical image data 150 and the constraint data and/or objective data; and
wherein the generating a first treatment plan comprises, for each of the beams 120 of therapeutic radiation, simultaneously optimizing a gating window 110 and a corresponding set of multi-leaf collimator leaf positions for delivering the beam of therapeutic radiation to the anatomical region 130 in the temporal sequence of images so as to meet the set of at least one constraint and/or objective and to maximize a duration of the gating window.

In this third aspect, the system 100 simultaneously optimizes the gating window and the corresponding set of multi-leaf collimator leaf positions for delivering the beam of therapeutic radiation to the anatomical region in the temporal sequence of images so as to meet the set of at least one constraint and/or objective and to maximize a duration of the gating window. Simultaneously optimizing these parameters provides a more optimal combination of the gating window, and the corresponding set of multi-leaf collimator leaf positions, over e.g. sequentially optimizing these parameters.

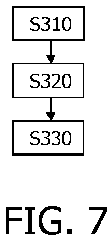

The operations performed by the system 100 in this third aspect are illustrated in Fig. 7, which is a flowchart illustrating an example of a computer-implemented method of generating a treatment plan for delivering a plurality of beams of therapeutic radiation to an anatomical region undergoing cyclical motion, in accordance with some aspects of the present disclosure. It is noted that operations described as being performed by the one or more processors 140 of the system 100 illustrated in Fig. 1 in relation to this third aspect, may also be performed in the method illustrated in Fig. 7. Likewise, operations described in relation to the method illustrated in Fig. 7, may also be performed by the one or more processors 140 of the system 100 illustrated in Fig. 1 as elements of this third aspect.

In this third aspect, the operation S310 of receiving medical image data 150, is performed in the same manner as described above in relation to the operation S110.

The constraint data that is received in the operation S320 may be generated automatically, or it may be generated in response to user input, as described above. The constraint data and/or objective data define a set of at least one constraint and/or objective for the anatomical region (130). The constraint data and/or objective data may define a set of at least one constraint and/or objective for the target anatomical region within the anatomical region (130). The constraint data may represent constraints such as the constraints described above. Likewise, the objective data that is received in the operation S320 may be generated automatically, or it may be generated in response to user input. The objective data may represent objectives such as requirements for the radiation dose delivered to parts of the anatomical region, which should be fulfilled. In contrast to the constraints, and which must be met, the objectives should be met as best as possible. For instance, a requirement specified by an objective of the radiation dose may include a minimum radiation dose to be delivered to a target anatomical region, for example.

In the operation S330, a first treatment plan is generated based on the medical image data 150 and the constraint data. Techniques used in various commercial software packages may be used for this purpose, including techniques used in Pinnacle, marketed by Philips Healthcare, Best, The Netherlands.

In this third aspect, the operation of simultaneously optimizing a gating window 110 and a corresponding set of multi-leaf collimator leaf positions, may include performing an inverse optimization procedure wherein the gating window and the corresponding set of multi-leaf collimator leaf positions are used to determine a spatial distribution of a dose delivered by the beam 120 of therapeutic radiation to the anatomical region 130 in the images in the temporal sequence corresponding to the gating window, and wherein the spatial distribution of the dose is used to determine whether the set of at least one constraint and/or objective is met.

In this third aspect, a second treatment plan may also be generated in which the corresponding gating window 110 has a predetermined duration, or in which the beam is delivered without gating. Thus, in one example, the one or more processors 140 are configured to:
generate a second treatment plan for delivering a plurality of beams 120 of therapeutic radiation the anatomical region 130 based on the medical image data 150 and the constraint and/or objective data; and
output one or more metrics for comparing the first treatment plan with the second treatment plan; and
wherein the generating a second treatment plan comprises, for each of the beams 120 of therapeutic radiation, optimizing a set of multi-leaf collimator leaf positions for delivering the beam of therapeutic radiation to the anatomical region 130 in the temporal sequence of images 160_{1..m} so as to meet the set of at least one constraint and/or objective; and
wherein the optimizing a set of multi-leaf collimator leaf positions for the second treatment plan is performed using a corresponding gating window 110 having a predetermined duration; or
wherein the optimizing a set of multi-leaf collimator leaf positions for the second treatment plan is performed without gating the beam 120 of therapeutic radiation.

By providing the second treatment plan, the system 100 facilitates a comparison between a treatment plan that includes gating, a treatment plan that is delivered with a specified gating window, and a treatment plan that is delivered without gating. This facilitates a physician to compare the different treatment plans.

In this example, the one or more metrics may comprise one or more of:
a graphical representation comprising a spatial distribution of a dose delivered to the anatomical region 130 by each of the first treatment plan and the second treatment plan; a graphical representation comprising a spatial distribution of a difference in the dose delivered to the anatomical region 130 by the first treatment plan and the second treatment plan;
an indication of a duration of the gating window 110 for the first treatment plan;
an indication of a size of the envelope 170 swept by the anatomical region 130 in the images corresponding to the gating window 110 for the first treatment plan;
an indication of a size of the envelope 170 swept by the anatomical region 130 in the images corresponding to a complete cycle of the cyclical motion;
an indication of a measure of a degree to which the at least one constraint and/or objective is met for each treatment plan;
a dose-volume histogram for an anatomical structure within the anatomical region 130.

In this third aspect, the system 100 may also provide a recommendation to deliver the first treatment plan, or to deliver the second treatment plan. Thus, in one example, the one or more processors 140 are further configured to:
output a recommendation to deliver the first treatment plan, or to deliver the second treatment plan, and wherein the recommendation is based on one of more of:
a spatial distribution of a dose delivered to the anatomical region 130 by each of the first treatment plan and the second treatment plan;
a duration of the gating window 110 for the first treatment plan;
a size of the envelope 170 swept by the anatomical region 130 in the images corresponding to the gating window 110 for the first treatment plan;
a size of the envelope 170 swept by the anatomical region 130 in the images corresponding to a complete cycle of the cyclical motion;
a measure of a degree to which the at least one constraint and/or objective is met for each treatment plan.

In this third aspect, various other information may be outputted by the one or more processors. For instance, the one or more processors 140 may be configured to output one or more of:
control parameters for controlling the beam 120 of therapeutic radiation in accordance with the generated treatment plan;
an indication of a duration of the gating window 110 for the first treatment plan;
an indication of a start Ampₛₜₐᵣₜ, Pₛₜₐᵣₜ and an end Amp_{end}, P_{end} of the gating window 110 in the cycle for the first treatment plan;
a graphical representation comprising a spatial distribution of a dose delivered to the anatomical region 130 by the treatment plan;
an indication of a total delivery time for delivering the treatment plan
an indication of a size of the envelope 170 swept by the anatomical region 130 in the images corresponding to the gating window 110 for the first treatment plan;
an indication of a size of the envelope 170 swept by the anatomical region 130 in the images corresponding to a complete cycle of the cyclical motion;
an indication of a measure of a degree to which the at least one constraint and/or objective is met for each treatment plan.

In this third aspect, it is noted that some parts of the first treatment plan may be delivered with gating, and that some parts of the first treatment plan may be delivered without gating. Thus, in one example, wherein the generating a first treatment plan comprises, for at least one of the beams 120 of therapeutic radiation:
simultaneously optimizing the gating window 110 and the corresponding set of multi-leaf collimator positions for delivering the beam 120 of therapeutic radiation to the anatomical region 130 in the temporal sequence of images 160_{1..m} so as to meet the set of at least one constraint and/or objective and to maximize the duration of the gating window and to deliver a first portion of a total radiation dose to the anatomical region; and
optimizing the set of multi-leaf collimator positions for delivering the beam 120 of therapeutic radiation to the anatomical region in the temporal sequence of images so as to meet the set of at least one constraint and/or objective and to deliver a second portion of the total radiation dose to the anatomical region 130; and
wherein the second portion of the total radiation dose is delivered without gating the beam 120.

The ability to deliver a treatment plan using a combination of gating, and without gating, facilitates the delivery of a treatment plan in a more time-efficient manner. For instance, some portions of the anatomy that undergo a relatively small amount of motion may be treated without gating, whereas other portions of the anatomy that undergo a relatively larger amount of motion are treated with gating..

In any of the three aspects described above, the one or more processors 140 may also output i) a recommendation to plan a gated delivery of a beam 120 of therapeutic radiation to the anatomical region 130, or ii) a recommendation to plan a non-gated delivery of a beam 120 of therapeutic radiation to the anatomical region, or iii) a recommended fractionation scheme for delivering a beam 120 of therapeutic radiation to the anatomical region, for each of the one or more gating windows 110, based on a comparison of a size of an envelope 170 swept by the anatomical region 130 in the images in the temporal sequence corresponding to the gating window, with a threshold value. These recommendations may be generated using various techniques, including for instance using decision tree, or a look-up table, or a trained neural network, that respectively relate the recommendation to the size of an envelope 170. These recommendations may be determined based on expert knowledge recorded in the decision tree, and so forth, as described above.

In any of the three aspects described above, the one or more processors 140 may also be configured to calculate a magnitude of a displacement of the anatomical region 130 between each pair of consecutive images in the images in the temporal sequence corresponding to each of the one or more gating windows 110. The one or more processors 140 may also be configured to output i) a recommendation to plan a gated delivery of a beam 120 of therapeutic radiation to the anatomical region, or ii) a recommendation to plan a non-gated delivery of a beam 120 of therapeutic radiation to the anatomical region, or iii) a recommended fractionation scheme for delivering a beam 120 of therapeutic radiation to the anatomical region, for each of the one or more gating windows, based on a comparison of a maximum displacement of the anatomical region 130 between the pairs of consecutive images in the images in the temporal sequence corresponding to the gating window, and a threshold value.

The maximum displacement of the anatomical region 130 between the pairs of consecutive images in the images in the temporal sequence may for instance be used to recommend a non-gated treatment if the maximum displacement of the anatomical region 130 between all pairs of consecutive images is below the threshold value for all phase values.

In any of the three aspects described above, the one or more processors 140 may be configured to:
output a graphical representation of a motion signal representing the cyclical motion of the anatomical region 130; and
map the outputted gating window 110 onto the graphical representation of the motion signal to define a start amplitude Ampₛₜₐᵣₜ and an end amplitude Amp_{end} of the motion signal for triggering the delivery of the beam of therapeutic radiation to the anatomical region 130.

The motion signal may include a discrete set of predefined phase intervals for delivering the beam 120 of therapeutic radiation to the anatomical region 130, or the motion signal comprises a discrete set of predefined amplitude intervals for delivering the beam of therapeutic radiation to the anatomical region. Thus, phase-gating, or amplitude-gating, may be used to deliver the beam to the anatomical region, or to trigger the acquisition of the medical images.

If predefined phase intervals, or predefined amplitude intervals, are used, an uncertainty value may also be generated for the displacement, or the size of the envelope, during the intervals. Thus, in one example, the one or more processors 140 are configured to:
output an uncertainty value representing a variation in the displacement of the anatomical region 130 during each of the outputted predefined phase intervals, or during each of the predefined amplitude intervals, respectively; and
wherein the uncertainty values are calculated based on a variation in the size of the envelope 170 swept by the anatomical region 130 throughout the images in the images in the temporal sequence that fall within each of the outputted predefined phase intervals, or within each of the predefined amplitude intervals, respectively.

In any of the three aspects described above, the one or more processors 140 may calculate a duty cycle for at least one of the outputted gating windows 110. The duty cycle is calculated based on a duration of the at least one outputted gating window as a proportion of a complete cycle of the cyclical motion. The one or more processors 140 may also output an indication of the duty cycle for the at least one outputted gating window. The duty cycle provides an indication of the efficiency with which the beam is delivered.

In any of the three aspects described above, the one or more processors 140 may calculate a size of an envelope 170 swept by the anatomical region 130 throughout the images corresponding to the at least one gating window 110, and output an indication of the size of the envelope 170 for the at least one gating window. The size of the envelope provides an indication of the amount of motion of the anatomical region during the gating window.

In any of the three aspects described above, the one or more processors 140 may receive input defining at least one user-selected start and end of a gating window 110 during the cycle of the cyclical motion, calculate a size of an envelope 170 swept by the anatomical region 130 throughout the images corresponding to the portion of the cycle defined by the at least one user-selected start and end of the gating window 110, and output an indication of the size of the envelope 170 for the at least one user-selected start and end of the gating window 110. Providing a user with the ability to define the gating window in this manner facilitates experimentation with different gating windows.

In any of the three aspects described above, the one or more processors 140 may calculate at least one therapeutic plan for delivering a beam 120 of therapeutic radiation to the anatomical region 130 based on the size of the envelope 170 swept by the anatomical region 130 throughout the images corresponding to at least one of the outputted gating windows 110. The therapeutic plan may be calculated using the techniques described above. The one or more processors may also be used to control the delivery of the therapeutic plan to the anatomical region 130.

It is noted that in the examples described above, the system 100 may also include one or more of: a medical imaging system for providing the medical image data 150, such as for example the CT imaging system 180 illustrated in Fig. 1; a monitor (not illustrated in Fig. 1) for displaying the temporal sequence of images 160_{1..m} generated by the medical imaging system, motion signals, indications of the gating windows, envelopes, and so forth; a video camera 190 for generating a motion signal representing the anatomical region; a radiation therapy delivery system 200 for delivering a treatment plan; a patient bed; and a user input device (not illustrated in Fig. 1) for receiving user input in relation to the operations performed by the system, such as a keyboard, a mouse, a touchscreen, and so forth.

It is noted that any of the aspects described above in relation to the system 100 may alternatively be provided in the form of a computer-implemented method, or a computer program product.

In one example, a computer-implemented method of determining a gating window 110 for delivering a beam 120 of therapeutic radiation to an anatomical region 130 undergoing cyclical motion, is provided. The method comprises:
receiving S 110 medical image data 150, the medical image data 150 comprising a temporal sequence of images 160_{1..m} representing the cyclical motion of the anatomical region 130;
determining S120, for each of one or more sets of at least one constraint imposed on the anatomical region, a maximum gating window 110 throughout which a position of the anatomical region 130 in the images 160_{1..m} in the temporal sequence satisfies the set of at least one constraint, and a corresponding start Ampₛₜₐᵣₜ, Pₛₜₐᵣₜ and end Amp_{end}, P_{end} of the gating window in the cycle; and
outputting S130 an indication of the one or more maximum gating windows 110, and for each maximum gating window, the corresponding start Ampₛₜₐᵣₜ, Pₛₜₐᵣₜ and end Amp_{end}, P_{end} of the gating window in the cycle.

In another example, a computer program product, is provided. The computer program product comprises instructions which when executed by one or more processors, cause the one or more processors to carry out a method of determining a gating window 110 for delivering a beam 120 of therapeutic radiation to an anatomical region 130 undergoing cyclical motion, is provided. The method comprises:
receiving S110 medical image data 150, the medical image data 150 comprising a temporal sequence of images 160_{1..m} representing the cyclical motion of the anatomical region 130;
determining S120, for each of one or more sets of at least one constraint imposed on the anatomical region, a maximum gating window 110 throughout which a position of the anatomical region 130 in the images 160_{1..m} in the temporal sequence satisfies the set of at least one constraint, and a corresponding start Ampₛₜₐᵣₜ, Pₛₜₐᵣₜ and end Amp_{end}, P_{end} of the gating window in the cycle; and
outputting S130 an indication of the one or more maximum gating windows 110, and for each maximum gating window, the corresponding start Ampₛₜₐᵣₜ, Pₛₜₐᵣₜ and end Amp_{end}, P_{end} of the gating window in the cycle.

In another example, a computer-implemented method of quantifying a displacement of an anatomical region 130 undergoing cyclical motion, is provided. The method comprises:
receiving S210 medical image data 150, the medical image data 150 comprising a temporal sequence of images 160_{1..m} representing the displacement of the anatomical region 130 during the cyclical motion;
determining S220, for each of one or more gating windows 110_{1..i} during a cycle of the cyclical motion, a corresponding start Ampₛₜₐᵣₜ₁, Pₛₜₐᵣₜ₁ and end Amp_{end1}, P_{end1} of the gating window 110 defining a portion of the cycle throughout which a size of an envelope 170 swept by the anatomical region 130 in the images in the temporal sequence is smallest for the duration of the gating window 110; and
outputting S230 an indication of the one or more gating windows 110_{1..i}, and for each gating window 110₁, an indication of the corresponding start Ampₛₜₐᵣₜ₁, Pₛₜₐᵣₜ₁ and end Amp_{end1}, P_{end1} of the gating window in the cycle and an indication of the size of the envelope 170 swept by the anatomical region 130.

In another example, a computer program product, is provided. The computer program product comprises instructions which when executed by one or more processors, cause the one or more processors to carry out a method of quantifying a displacement of an anatomical region 130 undergoing cyclical motion, is provided. The method comprises:
receiving S210 medical image data 150, the medical image data 150 comprising a temporal sequence of images 160_{1..m} representing the displacement of the anatomical region 130 during the cyclical motion;
determining S220, for each of one or more gating windows 110_{1..i} during a cycle of the cyclical motion, a corresponding start Ampₛₜₐᵣₜ₁, Pₛₜₐᵣₜ₁ and end Amp_{end1}, P_{end1} of the gating window 110 defining a portion of the cycle throughout which a size of an envelope 170 swept by the anatomical region 130 in the images in the temporal sequence is smallest for the duration of the gating window 110; and
outputting S230 an indication of the one or more gating windows 110_{1..i}, and for each gating window 110₁, an indication of the corresponding start Ampₛₜₐᵣₜ₁, Pₛₜₐᵣₜ₁ and end Amp_{end1}, P_{end1} of the gating window in the cycle and an indication of the size of the envelope 170 swept by the anatomical region 130.

In another example, a computer-implemented method of generating a treatment plan for delivering a plurality of beams 120 of therapeutic radiation to an anatomical region 130 undergoing cyclical motion, is provided. The method comprises:
receiving S310 medical image data 150, the medical image data 150 comprising a temporal sequence of images 160_{1..m} representing the cyclical motion of the anatomical region 130;
receiving S320 constraint data and/or objective data defining a set of at least one constraint and/or objective for the anatomical region 130; and
generating S330 a first treatment plan for the anatomical region 130 based on the medical image data 150 and the constraint data and/or objective data; and
wherein the generating a first treatment plan comprises, for each of the beams 120 of therapeutic radiation, simultaneously optimizing a gating window 110 and a corresponding set of multi-leaf collimator leaf positions for delivering the beam of therapeutic radiation to the anatomical region 130 in the temporal sequence of images so as to meet the set of at least one constraint and/or objective and to maximize a duration of the gating window.

In another example, a computer program product, is provided. The computer program product comprises instructions which when executed by one or more processors, cause the one or more processors to carry out a method of generating a treatment plan for delivering a plurality of beams 120 of therapeutic radiation to an anatomical region 130 undergoing cyclical motion, is provided. The method comprises:
receiving S310 medical image data 150, the medical image data 150 comprising a temporal sequence of images 160_{1..m} representing the cyclical motion of the anatomical region 130;
receiving S320 constraint data and/or objective data defining a set of at least one constraint and/or objective for the anatomical region 130; and generating S330 a first treatment plan for the anatomical region 130 based on the medical image data 150 and the constraint data and/or objective data; and
wherein the generating a first treatment plan comprises, for each of the beams 120 of therapeutic radiation, simultaneously optimizing a gating window 110 and a corresponding set of multi-leaf collimator leaf positions for delivering the beam of therapeutic radiation to the anatomical region 130 in the temporal sequence of images so as to meet the set of at least one constraint and/or objective and to maximize a duration of the gating window.

The above examples are to be understood as illustrative of the present disclosure, and not restrictive. Further examples are also contemplated. For instance, the examples described in relation to a system, may also be provided by a corresponding computer-implemented method, or by a corresponding computer program product, or by a corresponding computer-readable storage medium. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for determining a gating window (110) for delivering a beam (120) of therapeutic radiation to an anatomical region (130) undergoing cyclical motion, the system comprising one or more processors (140) configured to:
receive (S110) medical image data (150), the medical image data (150) comprising a temporal sequence of images (160_{1..m}) representing the cyclical motion of the anatomical region (130);
determine (S120), for each of one or more sets of at least one constraint imposed on the anatomical region, a maximum gating window (110) throughout which a position of the anatomical region (130) in the images (160_{1..m}) in the temporal sequence satisfies the set of at least one constraint, and a corresponding start (Ampₛₜₐᵣₜ, Pₛₜₐᵣₜ) and end (Amp_{end}, P_{end}) of the gating window in the cycle; and
output (S130) an indication of the one or more maximum gating windows (110), and for each maximum gating window, the corresponding start (Ampₛₜₐᵣₜ, Pₛₜₐᵣₜ) and end (Amp_{end}, P_{end}) of the gating window in the cycle.

2. The system according to claim 1, wherein the one or more processors (140) are further configured to:
calculate, for each of a plurality of pairs of images (160_{1..m}) in the temporal sequence, a displacement of the anatomical region (130) between the pair of images; and
wherein the one or more processors (140) are configured to determine the one or more maximum gating windows (110), using the calculated displacements.

3. The system according to claim 1 or claim 2, wherein the one or more processors (140) are configured to determine the one or more maximum gating windows (110) by:
determining a shape of an envelope (170) swept by the anatomical region (130) in the images (160_{1..m}) in the temporal sequence corresponding to the gating window (110); and
assessing the shape of the envelope with respect to the set of at least one constraint.

4. The system according to any one of claims 1-3, wherein the one or more processors (140) are configured to determine the one or more maximum gating windows (110), by:
selecting from the temporal sequence of images (160_{1..m}), a series of images having a maximum duration and throughout which the anatomical region (130) meets the set of at least one constraint, the maximum gating window (110) being defined based on a difference between a time of a first image in the series and a time of a last image in the series, and the corresponding start (Ampₛₜₐᵣₜ, Pₛₜₐᵣₜ) and end (Amp_{end}, P_{end}) of the maximum gating window (110) in the cycle being defined based on the time of the first image in the series and the time of the last image in the series.

5. The system according to any one of claims 1-4, wherein the one or more processors (140) are further configured to:
output i) a recommendation to plan a gated delivery of a beam (120) of therapeutic radiation to the anatomical region (130), or ii) a recommendation to plan a non-gated delivery of a beam (120) of therapeutic radiation to the anatomical region (130), or iii) a recommended fractionation scheme for delivering of a beam of therapeutic radiation to the anatomical region (130), for each set of at least one constraint, based on the at least one constraint and the maximum gating window (110).

6. A system (100) for quantifying a displacement of an anatomical region (130) undergoing cyclical motion, the system comprising one or more processors (140) configured to:
receive (S210) medical image data (150), the medical image data (150) comprising a temporal sequence of images (160_{1..m}) representing the displacement of the anatomical region (130) during the cyclical motion;
determine (S220), for each of one or more gating windows (110_{1..i}) during a cycle of the cyclical motion, a corresponding start (Ampₛₜₐᵣₜ₁, Pₛₜₐᵣₜ₁) and end (Amp_{end1}, P_{end1}) of the gating window (110) defining a portion of the cycle throughout which a size of an envelope (170) swept by the anatomical region (130) in the images in the temporal sequence is smallest for the duration of the gating window (110); and
output (S230) an indication of the one or more gating windows (110_{1..i}, and for each gating window (110₁), an indication of the corresponding start (Ampₛₜₐᵣₜ₁, Pₛₜₐᵣₜ₁) and end (Amp_{end1}, P_{end1}) of the gating window in the cycle and an indication of the size of the envelope (170) swept by the anatomical region (130).

7. The system according to claim 6, wherein the one or more processors (140) are further configured to:
calculate, for each of a plurality of pairs of images (160_{1..m}) in the temporal sequence, a displacement of the anatomical region (130) between the pair of consecutive images; and
wherein the one or more processors (140) are configured to determine the one or more gating windows (110_{1..i}), using the calculated displacements.

8. The system according to claim 6 or claim 7, wherein the one or more processors (140) are configured to determine the start (Ampₛₜₐᵣₜ, Pₛₜₐᵣₜ) and end (Amp_{end}, P_{end}) of the gating window (110) in the cycle for each of the one or more gating windows (110_{1..i}) by determining a shape of an envelope (170) swept by the anatomical region (130) throughout each of a plurality of selections of images in the temporal sequence corresponding to the gating window, and determining the start (Ampₛₜₐᵣₜ, Pₛₜₐᵣₜ) and end (Amp_{end}, P_{end}) of the gating window based on the selection of images for which a corresponding size of the envelope (170) is smallest.

9. The system according to any one of claims 6-8, wherein the one or more processors (140) are configured to determine the start (Ampₛₜₐᵣₜ, Pₛₜₐᵣₜ) and end (Amp_{end}, P_{end}) of the gating window (110) in the cycle for each of the one or more gating windows (110_{1..i}), by:
selecting from the temporal sequence of images (160_{1..m}), a series of images having a duration corresponding to the gating window (110) and throughout which a size of an envelope (170) swept by the anatomical region (130) is smallest for the duration of the gating window, the corresponding start (Ampₛₜₐᵣₜ, Pₛₜₐᵣₜ) and end (Amp_{end}, P_{end}) of the gating window in the cycle being defined based on a time of a first image in the series and a time of a last image in the series.

10. The system according to any one of claims 6-9, wherein the one or more processors (140) are further configured to:
receive constraint data defining a set of at least one constraint for the anatomical region (130); and
for each of the one or more gating windows (110_{1..i}):
evaluate a position of the anatomical region (130) with respect to the set of at least one constraint using the images in the temporal sequence corresponding to the gating window; and
output an indication of whether or not the set of at least one constraint is met.

11. The system according to any one of claims 6 - 10, wherein the one or more processors (140) are further configured to:
output i) a recommendation to plan a gated delivery of a beam (120) of therapeutic radiation to the anatomical region (130), or ii) a recommendation to plan a non-gated delivery of a beam (120) of therapeutic radiation to the anatomical region (130), or iii) a recommended fractionation scheme for delivering a beam (120) of therapeutic radiation to the anatomical region (130), for each of the one or more gating windows (110_{1..i}), based on the duration of the gating window and the size of the envelope (170) swept by the anatomical region (130).

12. A system (100) for generating a treatment plan for delivering a plurality of beams (120) of therapeutic radiation to an anatomical region (130) undergoing cyclical motion, the system comprising one or more processors (140) configured to:
receive (S310) medical image data (150), the medical image data (150) comprising a temporal sequence of images (160_{1..m}) representing the cyclical motion of the anatomical region (130);
receive (S320) constraint data and/or objective data defining a set of at least one constraint and/or objective for the anatomical region (130); and
generate (S330) a first treatment plan for the anatomical region (130) based on the medical image data (150) and the constraint data and/or objective data; and
wherein the generating a first treatment plan comprises, for each of the beams (120) of therapeutic radiation, simultaneously optimizing a gating window (110) and a corresponding set of multi-leaf collimator leaf positions for delivering the beam of therapeutic radiation to the anatomical region (130) in the temporal sequence of images so as to meet the set of at least one constraint and/or objective and to maximize a duration of the gating window.

13. The system according to any previous claim, wherein the one or more processors (140) are configured to output i) a recommendation to plan a gated delivery of a beam (120) of therapeutic radiation to the anatomical region (130), or ii) a recommendation to plan a non-gated delivery of a beam (120) of therapeutic radiation to the anatomical region, or iii) a recommended fractionation scheme for delivering a beam (120) of therapeutic radiation to the anatomical region, for each of the one or more gating windows (110), based on a comparison of a size of an envelope (170) swept by the anatomical region (130) in the images in the temporal sequence corresponding to the gating window, with a threshold value.

14. The system according to any one or claims 1 - 5, or any one of claims 10 - 13, wherein the set of at least one constraint, comprises one or more of:
a constraint requiring that a displacement of the anatomical region (130) remains less than a specified value;
a constraint requiring that the anatomical region (130) in the images in the temporal sequence remains within a specified boundary;
a constraint requiring that a specified portion of the anatomical region (130) remains within a specified boundary for a specified portion of the cycle;
a constraint requiring that the anatomical region (130) receives a specified dose from a specified dose distribution of the beam (120) of therapeutic radiation.

15. The system according to any previous claim, wherein the one or more processors (140) are further configured to:
output a graphical representation of a motion signal representing the cyclical motion of the anatomical region (130); and
map the outputted gating window (110) onto the graphical representation of the motion signal to define a start amplitude (Ampₛₜₐᵣₜ) and an end amplitude (Amp_{end}) of the motion signal for triggering the delivery of the beam of therapeutic radiation to the anatomical region (130).
